# DEMANDE DE BREVET EUROPEEN

(11) **EP 4 733 270 A1**
(43) Date de publication de la demande: **29.04.2026**
(21) Numéro de dépôt: 25211143.0
(22) Date de dépôt: 24.10.2025
(51) Int. Cl.: C02F 1/32, C02F 1/72, A61L 2/08, A61L 2/10, C02F 1/78

(54) **PURIFICATEUR D'EAU PAR SOURCE UV ET PHOTOCATALYSE**

(30) Priorité: 25.10.2024 FR 2411665
(71) Demandeur: Meudal, Nicolas, 22610 Pleubian (FR)
(72) Inventeur: Meudal, Nicolas, 22610 Pleubian (FR)
(74) Mandataire: Novagraaf International SA

(57) **Abrégé**

Purificateur d'eau (100) comprenant :
• une première source lumineuse UV (118),
• un mélangeur statique (120) comprenant un photocatalyseur et situé autour de la première source lumineuse UV (110),
• une deuxième source lumineuse UV (138) située autour de le mélangeur statique (120);
dans lequel au moins une de la première source lumineuse UV (118) et de la deuxième source lumineuse UV (138) émet une lumière d'une longueur d'onde déclenchant la production par le photocatalyseur de radicaux hydroxyles et/ou d'ozone dans l'eau.

## Description

La présente concerne le domaine des technologies de désinfection de l'eau, en particulier les systèmes de réacteurs UV.

### État de la technique

Les systèmes de désinfection par UV sont couramment utilisés pour traiter l'eau. Par exemple, le document EP2394963A1 divulgue un dispositif de stérilisation aux ultraviolets pour une eau extérieure. De plus, le document US 2017/0225971 décrit un appareil et un procédé pour le traitement de l'eau.

### Résumé de l'invention

La désinfection de l'eau est un enjeu dans de nombreux secteurs, notamment pour garantir la sécurité sanitaire. Les réacteurs UV traditionnels utilisent généralement une lampe UV pour désinfecter l'eau en la faisant passer à proximité de la source lumineuse. L'eau entre par une entrée, traverse le réacteur où elle est exposée à la lumière UV, puis sort par une sortie. Cette méthode repose sur l'utilisation de lampes UV pour éliminer les micro-organismes présents dans l'eau.

Les solutions actuelles présentent plusieurs inconvénients. Premièrement, l'efficacité de la désinfection UV dépend de la transparence de l'eau. Les liquides non transparents, comme le café, posent des défis importants car la lumière UV ne peut pas pénétrer efficacement. De plus, les gaines en quartz utilisées pour protéger les lampes UV sont fragiles et coûteuses, ce qui augmente les coûts d'entretien. Un autre inconvénient majeur est l'absence de désinfectant résiduel après le traitement UV, contrairement au chlore, ce qui signifie que l'eau peut être recontaminée après le traitement. Enfin, le nettoyage des lampes UV, qui peuvent s'encrasser avec le temps, nécessite souvent des interventions manuelles ou chimiques, ce qui complique l'entretien.

L'invention revendiquée est définie par les revendications annexées et concerne un purificateur d'eau comprenant :
- une première source lumineuse UV,
- un mélangeur statique comprenant un photocatalyseur et situé autour de la première source lumineuse UV,
- une deuxième source lumineuse UV située autour du mélangeur statique; dans lequel au moins une de la première source lumineuse UV et de la deuxième source lumineuse UV émet une lumière d'une longueur d'onde déclenchant la production par le photocatalyseur de radicaux hydroxyles et/ou d'ozone dans l'eau.

Par illumination UV de la première source lumineuse UV et/ou de la deuxième source lumineuse UV, des radicaux hydroxyles et/ou de l'ozone sont générés permettant une désinfection additionnelle de l'eau. La longueur d'onde d'une des deux sources lumineuses UV peut être inférieure à 387 nm, par exemple de 254 nm à 365 nm. Préférentiellement, la première source lumineuse UV et la deuxième source lumineuse UV illuminent simultanément un même volume d'eau ou flux d'eau, par exemple présent ou circulant dans le mélangeur statique. La première source lumineuse UV peut être placée en regard de la deuxième source lumineuse UV, sans barrière opaque entre les sources lumineuses. Par exemple, les sources lumineuses sont disposées de manière concentrique ou co-axial l'une par rapport à l'autre.

Avantageusement, l'une de la première source lumineuse UV et de la deuxième source lumineuse UV est configurée pour émettre des UV-A et l'autre de la première source lumineuse UV et de la deuxième source lumineuse UV est configuré pour émettre des UV-C. Cette configuration permet une désinfection complémentaire par deux longueurs d'onde différentes, en plus de la génération de radicaux hydroxyles par le photocatalyseur, augmentant l'efficacité globale du système. En outre, l'illumination UV-C peut générer de l'ozone à partir de l'oxygène dissous présents dans l'eau à purifier.

Avantageusement, la première source de lumière UV est configurée pour émettre des UV-C. Cela assure une désinfection rapide et efficace des micro-organismes tout en assurant un haut niveau de sécurité pour les personnels évoluant autour du purificateur d'eau. Préférentiellement, la première source UV comprend des bandes ou de rubans de LED, par exemple arrangés sous forme de cylindre ou de tube.

Avantageusement, la deuxième source de lumière est configurée pour émettre des UV-A. Cela peut favoriser la génération de radicaux hydroxyles par le photocatalyseur tout en assurant un haut niveau de sécurité pour les personnels évoluant autour du purificateur d'eau. Préférentiellement, la deuxième source lumineuse UV comprend des bandes ou des rubans de LED.

Avantageusement, le purificateur d'eau comprend un premier corps transparent et un deuxième corps transparent contenant le premier corps transparent et le mélangeur statique, la première source lumineuse UV étant reçue dans le premier corps transparent, la deuxième source lumineuse UV étant placée à l'extérieur du deuxième corps transparent. Cette structure permet une protection et une efficacité maximales des sources lumineuses. Par exemple, le premier corps transparent peut faire partie de la première source lumineuse UV, par exemple dans le cas d'un tube de LED.

Avantageusement, le purificateur d'eau comprend un moteur agencé pour mettre en mouvement le mélangeur statique, le mélangeur statique étant en contact avec une paroi du premier corps transparent et/ou du deuxième corps transparent, le mélangeur statique étant configuré pour permettre par son mouvement un nettoyage de la paroi avec laquelle il est en contact. Cela assure un entretien automatique et réduit l'encrassement des surfaces. Par exemple, le mélangeur statique peut comprendre une pluralité de fils, de mousses, de lèvres en élastomère et/ou de racloirs permettant de nettoyer la paroi avec laquelle il est en contact.

Avantageusement, le mélangeur statique comprend au moins un conduit de distribution de produit nettoyant agencé pour distribuer le produit nettoyant dans le purificateur d'eau et connecté à une entrée de produit nettoyant. Cela permet une distribution uniforme du produit nettoyant, améliorant l'efficacité du nettoyage. Par exemple, le mélangeur statique peut être partiellement ou substantiellement creux et comprendre une pluralité de trou de petites tailles pour permettre la distribution du produit nettoyant au sein du purificateur d'eau.

Avantageusement, le purificateur d'eau comprend un axe longitudinal, une entrée d'eau à traiter et une sortie d'eau traitée opposée, dans lequel au moins une de l'entrée d'eau à traiter et de la sortie d'eau à traiter est désaxée par rapport à l'axe longitudinal. Cette disposition favorise un écoulement turbulent, améliorant le temps de contact et le mélange des fluides.

Avantageusement, le mélangeur statique comprend une pluralité de surfaces disposées de manière oblique par rapport à l'axe longitudinal du purificateur d'eau. Cela optimise le mélange et le temps de contact à l'intérieur du réacteur tout en offrant une surface accrue pour le photocatalyseur. Le mélangeur statique peut comprendre un élément avec plusieurs surfaces ou bien une pluralité d'éléments chacun avec une ou plusieurs surfaces.

Avantageusement, le purificateur d'eau comprend une entrée de produit désinfectant située du côté de l'entrée des effluents à traiter du purificateur d'eau. Cela permet une introduction efficace de produit désinfectant, augmentant l'efficacité du traitement, par exemple dans le cas d'une eau très contaminée.

Un deuxième aspect de l'invention concerne un procédé de purification d'eau avec un purificateur d'eau selon le premier aspect de l'invention comprenant les étapes suivantes :
- Faire circuler une eau à purifier dans le purificateur d'eau, préférentiellement dans une seule direction
- Illuminer l'eau à purifier avec la première source lumineuse UV et la deuxième source lumineuse UV, par exemple de manière simultanée.

Un troisième aspect de l'invention concerne l'utilisation d'un purificateur d'eau selon le premier aspect de l'invention pour purifier une eau.

### Description des figures

[FIG. 1] illustre un schéma en éclaté du purificateur d'eau avec un mélangeur statique et des sources lumineuses UV.
[FIG. 2] montre un schéma du réacteur du purificateur d'eau avec le mélangeur statique et les sources lumineuses UV assemblés.

### Description détaillée

La présente invention est dédiée à la purification d'une eau, par exemple une eau destinée à la consommation humaine ou animale, à une installation industrielle, à une installation agricole ou encore une eau destinée à être rejetée dans un milieu naturel. L'eau à purifier n'est pas nécessairement une eau pure mais peut comprendre de la matière en suspension, des sels dissous, des additifs alimentaires, des engrais ou encore des additifs industriels.

Dans un mode de réalisation général, le purificateur d'eau comprend une première source lumineuse UV par exemple en partie centrale du purificateur d'eau, entourée d'un élément d'agitation statique revêtu d'un photocatalyseur comme le TiO₂ ou le ZnO. Cet élément d'agitation statique est conçu pour maximiser la surface et le contact entre l'eau et la lumière UV, améliorant ainsi l'efficacité de la désinfection. En outre, le photocatalyseur est prévu pour générer des radicaux hydroxyles par illumination UV. Autour de cet élément, une deuxième source lumineuse UV peut renforcer le processus de photocatalyse.

Les ultraviolets (UV) sont des rayonnements électromagnétiques situés entre la lumière visible et les rayons X dans le spectre électromagnétique. Ils sont classés en trois catégories principales : UV-A, UV-B et UV-C, en fonction de leur longueur d'onde.

Les UV-A, avec des longueurs d'onde comprises entre 320 et 400 nanomètres, représentent environ 95 % des UV qui atteignent la surface terrestre. Ils pénètrent profondément dans la peau et sont principalement responsables du vieillissement cutané et des rides. Les UV-A peuvent être produits artificiellement à l'aide de lampes à vapeur de mercure, de lampes fluorescentes spéciales ou de LED UV-A.

Les UV-C, avec des longueurs d'onde comprises entre 100 et 280 nanomètres, sont les plus énergétiques et les plus dangereux des UV. Les UV-C sont utilisés principalement pour leurs propriétés germicides, car ils peuvent détruire les micro-organismes en endommageant leur ADN. Ils sont produits artificiellement à l'aide de lampes à mercure basse pression ou de LED UV-C mais sont dangereux pour les animaux et les êtres humains, notamment en cas d'exposition directe.

Un mélangeur statique est un dispositif couramment utilisé dans le traitement de l'eau pour mélanger efficacement des fluides ou des substances sans utiliser de pièces mobiles. Il se compose d'un ou plusieurs éléments fixes qui créent des turbulences contrôlées lorsque l'eau traverse le dispositif.

Par exemple, des surfaces présentant des profils obliques par rapport au flux d'eau à purifier sont disposés dans le purificateur d'eau entre la première source UV et la deuxième source UV, sans laisser de cheminement préféré au flux d'eau à purifier, imposant ainsi des cheminements tortueux et divisant et recombinant continuellement le flux d'eau. Le mélangeur statique peut être fabriqué en polymère, en céramique, en matériaux composite ou en métal inoxydable.

Dans la présente invention, le mélangeur statique est recouvert ou intègre un photocatalyseur, le photocatalyseur étant adapté pour générer des radicaux hydroxyles et/ou de l'ozone dans l'eau sous une illumination UV. Ce photocatalyseur est par exemple de l'oxyde de titane (TiO₂) ou de l'oxyde de zinc (ZnO). Ce photocatalyseur peut se présenter sous la forme d'un revêtement, de plaques fixées sur le mélangeur statique ou encore être intégré dans le corps du mélangeur statique, par exemple dans le cas d'un composite.

Le mélangeur statique peut ainsi à la fois augmenter le temps de contact de l'eau à purifier au sein du purificateur, contribuer à la désinfection par la génération de radicaux hydroxyles et/ou d'ozone et aussi assurer à ces radicaux ou à l'ozone une bonne homogénéisation dans le flux d'eau à purifier.

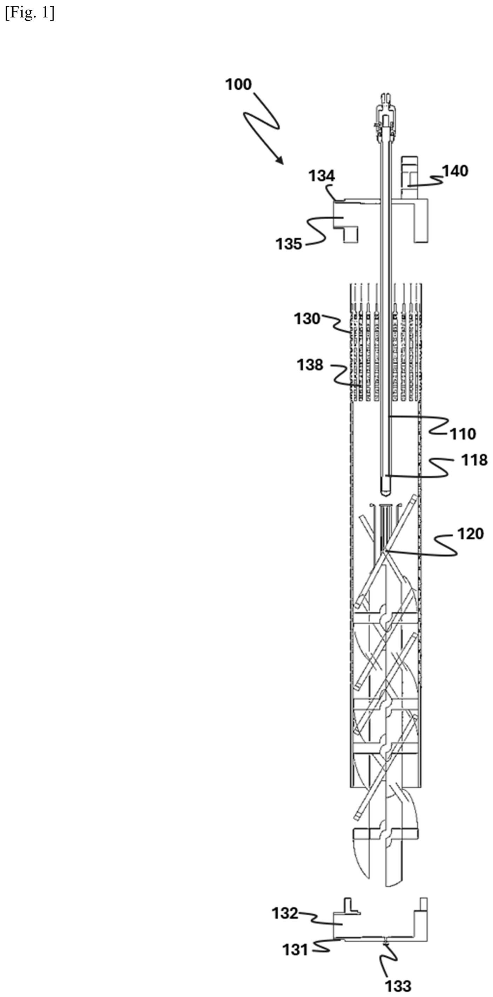

Dans l'exemple de la figure 1, le purificateur d'eau 100 comprend de l'intérieur vers l'extérieur, le premier corps transparent 110 accueillant ou intégrant la première source lumineuse UV 118, le mélangeur statique 120, le deuxième corps transparent 130 et la deuxième source lumineuse UV 138. Le deuxième corps transparent 130 peut servir de corps au purificateur d'eau 100. En outre, un boitier opaque non représenté peut recouvrir le tout ou au moins les deuxième sources lumineuses UV 138. Le deuxième corps transparent 130 peut être disposé autour ou de manière à englober le premier corps transparent 110. Les premier et deuxième corps transparents 110, 130 peuvent être des cylindres, par exemples disposés selon un même axe central, comme représenté sur la Fig. 1. Préférentiellement, aucune paroi opaque n'est placée entre la première source lumineuse UV et la deuxième source lumineuse UV.

Aux extrémités du deuxième corps transparent se trouve un capot d'entrée 131 pourvu d'une entrée d'eau 132 et optionnellement d'une entrée de produit désinfectant 133 et à l'opposée un capot de sortie 134 comprenant une sortie d'eau 135. Le premier corps transparent 110 et/ou la première source lumineuse UV 118 peut faire protrusion du capot de sortie de manière à permettre la connexion de la première source lumineuse UV 118 avec une alimentation électrique.

En outre, un moteur 140 peut être connecté au mélangeur statique 120 de manière à permettre un mouvement au sein du deuxième corps transparent 130, par exemple un mouvement de rotation.

La première source lumineuse UV émet préférentiellement des UV-C et la deuxième source lumineuse UV émet préférentiellement des UV-A. Ainsi, les UV-A peuvent générer les radicaux hydroxyles et/ou l'ozone par photocatalyse alors que les UV-C peuvent contribuer à la désinfection de l'eau par destructions de bactéries.

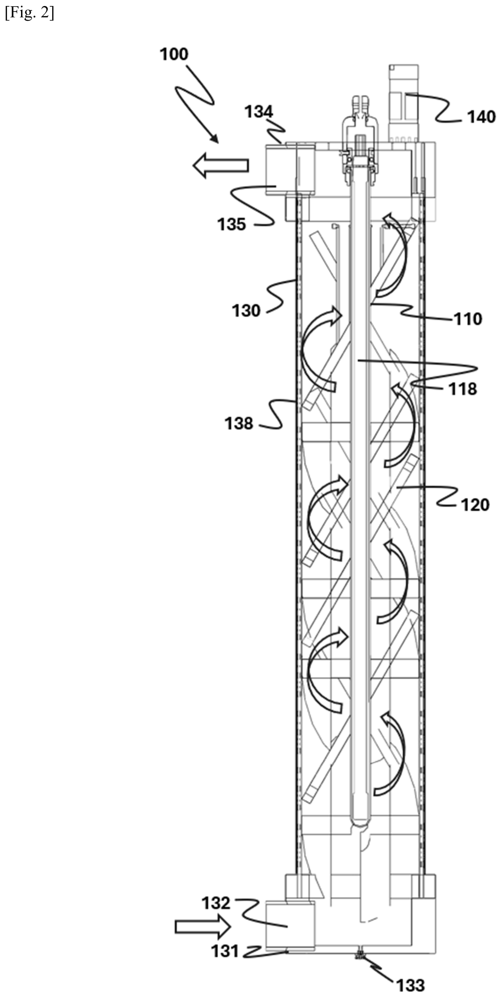

La Fig. 2 montre le purificateur d'eau 100 dans une configuration assemblée la première source lumineuse UV 118 étant alors placée en regard de la deuxième source lumineuse UV 138. En fonctionnement, l'eau à purifier entre par l'entrée d'eau 132 et circule entre le premiers corps transparent 110 et le deuxième corps transparent 130, le flux étant contrarié par le mélangeur statique 120 imposant une multitude de flux entre ses surfaces. La double illumination UV simultanée par les deux sources lumineuses UV permet à la fois une désinfection de l'eau, même en cas de matière en suspension, et la génération de radicaux hydroxyles et/ou d'ozone, permettant ainsi une double désinfection.

En sortie du purificateur d'eau, c'est-à-dire lors qu'elle passe par la sortie d'eau 135, l'eau est ainsi convenablement purifiée, sans nécessiter un volume de purificateur d'eau trop important ou une puissance électrique couteuse. Préférentiellement, l'eau fait un seul passage et/ou circule dans une seule direction à l'intérieur du purificateur d'eau 100, réduisant ainsi les pertes de charge.

Dans le cas d'une eau très contaminée, un produit désinfectant, par exemple à base de chlore, peut être introduit par l'entrée en produit désinfectant 133, procédant alors à une triple désinfection de l'eau dans le purificateur d'eau. Préférentiellement, aucune injection d'ozone est réalisée dans le purificateur d'eau 100.

De manière préférentielle, le purificateur d'eau 100 peut être configuré avec une entrée d'eau 132 et/ou une sortie d'eau 135 désaxées par rapport à un axe longitudinal du purificateur d'eau 100, créant un effet tourbillonnant qui optimise le mélange et le temps de contact de l'eau à purifier. Selon un plan médian longitudinal du purificateur d'eau, l'entrée d'eau 132 et/ou la sortie d'eau 135 peut ne pas être alignée avec ce plan médian.

Lors d'une opération d'entretien, le mélangeur statique, immobile en fonctionnement, peut être mis en rotation pour nettoyer par effet mécanique la paroi du premier corps transparent 110 et/ou du deuxième corps transparent 130 grâce au moteur 140. Ainsi, un nettoyage mécanique d'au moins une des parois internes du purificateur d'eau peut être réalisé simplement ou même automatiquement.

De manière préférentielle, le purificateur d'eau peut permettre l'addition d'un produit nettoyant, par exemple un produit tensioactif. Au moins une partie des éléments du mélangeur statiques peuvent ainsi être creux et pourvus de trous ou bien comprendre une pluralité de conduits (non-représentés) permettant une distribution du produit désinfectant directement sur les surfaces du mélangeur statique, par exemple à proximité ou dans les zones de contact avec la ou les parois à nettoyer. Cette amélioration permet ainsi un double nettoyage chimique et mécanique du purificateur d'eau, sans nécessiter de démontage complexe et long.

Enfin, un produit nettoyant peut en outre être introduit par l'entrée de produit désinfectant 133 ou une autre entrée, préférentiellement située dans l'axe du deuxième corps transparent et/ou de matière orthogonale à l'entrée d'eau 132, permettant encore de renforcer le nettoyage chimique du purificateur d'eau.

Le mélangeur statique peut s'étendre sur la totalité ou au moins la majeure partie de la longueur du deuxième corps transparent. Le premier corps transparent peut s'étendre sur la totalité ou au moins sur 80 % de la longueur du deuxième corps transparent.

Les entrées et sorties d'eau sont représentées sur les figures de manière orthogonales par rapport à un axe longitudinal du purificateur d'eau mais peuvent aussi être alignées avec cet axe. L'entrée d'eau et la sortie d'eau peuvent être inversées, ainsi l'eau peut entrer dans le purificateur d'eau par le dessus et sortir par le dessous. Alternativement, le purificateur d'eau peut être utilisé couché, c'est-à-dire avec son axe longitudinal orienté horizontalement.

Le purificateur d'eau peut être muni de tout type de vanne et de capteur usuel pour un purificateur d'eau et par exemple un capteur de débit, de température, de pression, de pH, de transmittance et de luminosité.

On comprendra que diverses modifications et/ou améliorations évidentes pour l'homme du métier peuvent être apportées aux différents modes de réalisation de l'invention décrits dans la présente description sans sortir du cadre de l'invention défini par les revendications annexées.

## Revendications

1. Purificateur d'eau (100) comprenant :
• une première source lumineuse UV (118),
• un mélangeur statique (120) comprenant un photocatalyseur et situé autour de la première source lumineuse UV (110),
• une deuxième source lumineuse UV (138) située autour du mélangeur statique (120);
dans lequel au moins une de la première source lumineuse UV (118) et de la deuxième source lumineuse UV (138) est adaptée pour émettre une lumière d'une longueur d'onde déclenchant la production par le photocatalyseur de radicaux hydroxyles et/ou d'ozone dans l'eau ;
**caractérisé en ce que** la première source lumineuse UV (118) et la deuxième source lumineuse UV (138) sont arrangées pour illuminer simultanément un même volume d'eau ou flux d'eau.

2. Purificateur d'eau (100) selon la revendication précédente, dans lequel une de la première source lumineuse UV (118) et de la deuxième source lumineuse UV (138) est configurée pour émettre des UV-A et l'autre de la première source lumineuse UV (118) et de la deuxième source lumineuse UV (138) est configurée pour émettre des UV-C.

3. Purificateur d'eau (100) selon l'une des revendications précédentes, dans lequel la première source lumineuse UV (118) est configurée pour émettre des UV-C et dans lequel la deuxième source lumineuse UV (138) est configurée pour émettre des UV-A.

4. Purificateur d'eau (100) selon l'une des revendications précédentes, comprenant un premier corps transparent (110) et un deuxième corps transparent (130) contenant le premier corps transparent (110) et le mélangeur statique (120), la première source lumineuse UV (118) étant reçue dans le premier corps transparent (110), la deuxième source lumineuse UV (138) étant placée à l'extérieur du deuxième corps transparent (130).

5. Purificateur d'eau (100) selon la revendication précédente, comprenant un moteur (140) agencé pour mettre en mouvement le mélangeur statique (120), le mélangeur statique (120) étant en contact avec une paroi du premier corps transparent (110) et/ou du deuxième corps transparent (130), le mélangeur statique (120) étant configuré pour permettre par son mouvement un nettoyage de la paroi du premier corps transparent (110) et/ou du deuxième corps transparent (130) avec laquelle il est en contact.

6. Purificateur d'eau (100) selon l'une quelconque des revendications précédentes, dans lequel le mélangeur statique (120) comprend au moins un conduit de distribution de produit nettoyant agencé pour distribuer un produit nettoyant dans le purificateur d'eau (100) et connecté à une entrée de produit nettoyant.

7. Purificateur d'eau (100) selon l'une des revendications précédentes, comprenant un axe longitudinal, une entrée d'eau (132) à traiter et une sortie d'eau (135) traitée opposée, dans lequel au moins une de l'entrée d'eau (132) et de la sortie d'eau (135) est désaxée par rapport à l'axe longitudinal.

8. Purificateur d'eau (100) selon la revendication précédente, le mélangeur statique (120) comprenant une pluralité de surfaces disposées de manières obliques par rapport à l'axe longitudinal.

9. Purificateur d'eau (100) selon l'une des revendications 7 et 8, comprenant en outre une entrée de produit désinfectant située du côté de l'entrée d'eau (132).

10. Procédé de purification d'eau avec un purificateur d'eau (100) selon l'une quelconque des revendications précédentes, comprenant les étapes suivantes :
a. Faire circuler une eau à purifier dans le purificateur d'eau (100),
b. Illuminer l'eau à purifier avec la première source lumineuse UV (118) et la deuxième source lumineuse UV (138).
